# EUROPEAN PATENT APPLICATION

(11) **EP 3 213 786 A1**
(43) Date of publication of application: **06.09.2017**
(21) Application number: 15854116.9
(22) Date of filing: 30.10.2015
(51) Int. Cl.: A61M 5/28, A61M 5/32

(54) **PRE-FILLED SYRINGE PREPARATION WITH NEEDLE, WHICH IS EQUIPPED WITH SYRINGE CAP**

(30) Priority: 30.10.2014 JP 2014221127
(71) Applicant: Chugai Seiyaku Kabushiki Kaisha, Kita-ku Tokyo 115-8543 (JP)
(72) Inventor: SHIOZAKI, Rieko, Tokyo 115-8543 (JP); YAMANAKA, Yuji, Tokyo 115-8543 (JP); TAKAHASHI, Mika, Tokyo 115-8543 (JP); YAMAZAKI, Tadao, Tokyo 115-8543 (JP); FUKUDA, Masakazu, Tokyo 115-8543 (JP); YAMASHITA, Shogo, Tokyo 115-8543 (JP)
(74) Representative: Vossius & Partner Patentanwälte Rechtsanwälte mbB
(86) International application number: PCT/JP2015/080834
(87) International publication number: WO 2016/068333

(57) **Abstract**

The purpose of the present invention is to provide a pre-filled syringe formulation with staked needle, which contains an antibody at a high concentration and can be produced on an industrial scale in a simple manner without causing clogging. A pre-filled syringe formulation with staked needle, in which a protein solution is packed. The pre-filled syringe formulation is characterized in that a cap formed with a material having low water vapor permeability is provided at the needle.

## Description

### Technical Field

The present invention relates to syringe caps for protecting a needle, syringes with staked needle, pre-filled syringe formulation, and methods of manufacturing pre-filled syringe formulation with staked needle on an industrial scale.

### Background Art

Various antibody-containing formulations have been developed and practically used in recent years. Many antibody-containing formulations are used as formulated solutions for intravenous injection. On the other hand, there have been growing demands to develop antibody-containing formulations as self-injectable formulated solutions for subcutaneous injection because of the needs in medical facilities. Formulated solutions filled in a pre-filled syringe have especially been demanded to be developed because of their convenience.

In designing antibody-containing formulations for subcutaneous injection, the concentration of the antibody in the liquid to be administered needs to be high, because the amount of antibody in a single dose is large (i.e., about 80 to 200 mg) but typical subcutaneous injections have a limitation on the volume of the liquid to be injected.

In recent years, as pre-filled syringe formulation for self-injection, pre-filled syringes having a cylindrical syringe body filled with a drug, a needle attached at the tip of the syringe body, and a plunger which is put in the syringe body and is slidable along the axis of the syringe body, have increasingly been used in medical facilities. Typically, this type of the pre-filled syringes further include a syringe cap which is removably attached to the syringe body and covers the needle, to thereby prevent accidental pricking of a hand or a finger of a user with the needle before the use of a pre-filled syringe.

For glass syringes for being filled by a tray filler system, sterilization before the filling of pharmaceutical liquid is performed using a gas capable of killing bacteria, such as ethylene oxide. The reason for this lies in the fact that glass is turned to blown when exposed to radiation such as a gamma ray. Accordingly, caps for syringes with staked needle are made of a gas-permeable material to allow gases capable of killing bacteria to pass through the cap and reach the needle.

As rubber plugs which are used for pre-filled syringes and made of a material that is poorly permeable to gas, such as a butyl rubber, examples include tip caps for pre-filled syringes described in Japanese Patent No. 4586079. All of the described syringes are needless glass syringes which are washed, sterilized, and assembled in factories. None of the rubber plugs is used as a needle cap for pre-filled syringes with staked needle as in the case of the present invention.

### Citation List

### Patent Literature

PTL 1: Japanese Patent No. 4586079

### Summary of Invention

### Technical Problem

For glass syringes for being filled by a tray filler system, sterilization before the filling of pharmaceutical liquid is performed using a gas capable of killing bacteria such as ethylene oxide. The reason for this lies in the fact that glass is turned to blown when exposed to radiation such as a gamma ray. Accordingly, caps for syringes with staked needle are made of a gas-permeable material to allow gases capable of killing bacteria to pass through it and reach the needle.

On the other hand, to design antibody-containing formulations for pre-filled syringes (PFS) for subcutaneous injection, it is essential to increase the concentration of the antibody in the liquid to be administered because the amount of antibody in a single dose is large (i.e., about 80 to 200 mg) but typical subcutaneous injections have a limitation on the volume of the liquid to be injected.

The present inventors found that, in the case of a formulated solution containing an antibody at such a high concentration in a pre-filled syringe with staked needle, when the pre-filled syringe is left for a long time under a dry condition without being packaged with, for example, a film or after having been taken from a package and if its needle cap is made of a material having a gas permeability, the formulated solution in the needle is dried and can cause clogging of the needle.

### Solution to Problem

The present inventors then found that clogging of pre-filled syringe (PFS) assemblies with staked needle can be prevented by using a needle cap made of a material that is poorly permeable to gas, such as a butyl rubber.

On the other hand, for glass syringes for being filled by a tray filler system used in the production on an industrial scale, such needle caps that are poorly permeable to gas make it difficult to gas-sterilize the tip of a needle of the glass syringes for being filled by a tray filler system. With this respect, the present inventors found that, by using a resin that will not change in color when exposed to radiation for sterilization as a material for syringes for being filled by a tray filler system, it was possible to provide pre-filled syringe formulation with staked needle containing an antibody at a high concentration, which could easily be manufactured on an industrial scale and whose needle would not be clogged.

More specifically, the present invention provides the following [1] to [14].
[1] A pre-filled syringe formulation with staked needle, the pre-filled syringe formulation being filled with a protein solution and sealed, including: a cap on the needle, the cap being made of a material having a low water vapor permeability.
[2] The pre-filled syringe formulation described in [1], wherein the material of the cap is a butyl rubber.
[3-1] The pre-filled syringe formulation in [2], wherein the butyl rubber is a n-butyl rubber or a halogenated butyl rubber.
[3-2] A pre-filled syringe formulation with staked needle, the pre-filled syringe formulation being filled with a protein solution and sealed, including: a cap on the needle, the cap being made of a material having a low water vapor permeability, wherein the cap is made of a material having a water vapor permeability of 0.1 g/m²·day or lower, or 0.05 g/m²·day or lower, or 0.01 g/m²·day or lower at 5°C; or 0.2 g/m²·day or lower, or 0.1 g/m²·day or lower at 25°C; or 0.2 g/m²·day or lower, or 0.1 g/m²·day or lower at 40°C (according to JIS 7126-1).
[3-3] The pre-filled syringe formulation described in [3-2], wherein the cap is made of a material having a water vapor permeability of 0.01 g/m²·day or lower (e.g., about 0.006 g/m²·day) at 5°C; or 0.1 g/m²·day or lower (e.g., about 0.072 g/m²·day) at 25°C; or 0.1 g/m²·day or lower (e.g., about 0.081 g/m²·day) at 40°C.
[3-4] The pre-filled syringe formulation described in [3-2] wherein the cap is made of a material having a water vapor permeability of 0.01 g/m²·day or lower (e.g., about 0.006 g/m²·day) at 5°C; and 0.1 g/m²·day or lower (e.g., about 0.072 g/m²·day) at 25°C; and 0.1 g/m²·day or lower (e.g., about 0.081 g/m²·day) at 40°C.
[4] The pre-filled syringe formulation in [1] to [3], wherein a syringe body is made of a resin.
[5] The pre-filled syringe formulation in [4], wherein the syringe body is made of a cycloolefinic resin.
[6] The pre-filled syringe formulation in [5], wherein the cycloolefinic resin is COP or COC.
[7] The pre-filled syringe formulation described in [1] to [6], wherein the protein solution comprises a protein at 80 mg/ml or more.
[8] The pre-filled syringe formulation in [7], wherein the protein solution is comprises the protein at 100 mg/ml or more.
[9] The pre-filled syringe formulation described above, wherein the protein solution has a viscosity of 2 to 100 mPa·s.
[10] The pre-filled syringe formulation described above, wherein the protein is an antibody.
[11] The pre-filled syringe formulation described above, wherein a concentration of the antibody is 100 to 300 mg/mL, and the viscosity is 6 to 100m Pa·s.
[12] The pre-filled syringe formulation described in [10] or [11], wherein the antibody is an anti-IL6 receptor antibody.
[13] The pre-filled syringe formulation in [12], wherein the antibody is tocilizumab.
[14] A method of producing a pre-filled syringe formulation with staked needle, the pre-filled syringe formulation being filled with a protein solution and sealed, the method including the steps of:
   1) sterilizing a syringe with staked needle with radiation or an electron beam, the syringe comprising a cap made of a material having a low water vapor permeability; and
   2) filling, with a protein solution, and sealing the syringe with staked needle aseptically.

### Advantageous Effects of Invention

According to the pre-filled syringe formulation of the present invention, it is possible to prevent clogging of PFS formulations by using a needle cap made of a material which is substantially impermeable to water vapor.

Furthermore, by using a resin that will not change in color when exposed to radiation for sterilization as a material for syringes for being filled by a tray filler system, it is possible to provide pre-filled syringe formulation with staked needle containing an antibody at a high concentration, which can easily be manufactured on an industrial scale without causing clogging.

### Brief Description of Drawings

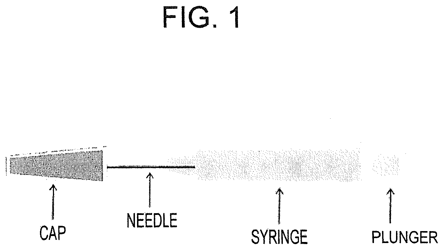

[Fig. 1] Fig. 1 is a diagrammatic representation for explaining a pre-filled syringe with staked needle with a needle cap (before being filled with pharmaceutical liquid) according to the present invention.

### Description of Embodiments

In the present invention, a "cap" refers to a "needle cap" or a "syringe cap" directed to protect a needle of a pre-filled syringe formulation and cover it aseptically. These three terms are interchangeably used herein depending on the context. A cap is removably attached to a syringe body so that it can be attached to a syringe body in the manufacture of the syringe formulation and removed therefrom before the use of the syringe formulation. Furthermore, the cap is cohesively attached to a syringe body or a connecter between the needle and the syringe body so that the needle is sealed.

An example of a pre-filled syringe with staked needle with a needle cap according to the present invention is shown in Fig. 1.

The pre-filled syringe formulation with staked needle with a needle cap according to an embodiment of the present invention is described below.

Needled syringes according to the present invention are characterized by having a syringe body in which a drug can be filled, a needle attached to the tip of the syringe body, and a syringe cap removably attached to the syringe body, which is poorly permeable to gas and covers the needle. With this configuration, clogging of the pre-filled syringes that have been left in a dry condition for a long time can be prevented.

Clogging of the pre-filled syringe is a phenomenon where pharmaceutical liquid is dried in the needle and becomes difficult to be discharged upon use. Accordingly, to prevent clogging, it is preferable that at least a portion of the syringe cap (i.e., needle cap) which covers the tip of the needle is made of a material with a low gas permeability, especially a low water vapor permeability.

The water vapor permeability can be assessed using a known test method. For example, the water vapor permeability can be represented as a mass of water vapor transmitted through a unit area of a specimen in a unit time under specified conditions of temperature and humidity. Examples of known standards include Japanese Industrial Standards (JIS) K 7126-1:2006, "Plastics -- Film and sheeting -- Determination of gas-transmission rate -- Part 1: Differential-pressure method," and ISO 2528 "Sheet materials -- Determination of water vapour transmission rate -- Gravimetric (dish) method."

For example, the water vapor permeability of the syringe cap used in the present invention is, at 5°C for example, preferably 0.1 g/m²·day or lower, or 0.05 g/m²·day or lower, or 0.01 g/m²·day or lower; at 25°C, preferably 0.2 g/m²·day or lower or 0.1 g/m²·day or lower; or at 40°C, preferably 0.2 g/m²·day or lower or 0.1 g/m²·day or lower, under pharmaceutical storage conditions according to JIS 7126-1:2006.

Or, it is preferable that the water vapor permeability is low in a temperature range (from about 5°C to about 40°C) under storage conditions from the time of manufacture of a pre-filled syringe to the time of its use. It is more preferable that the water vapor permeability is 0.1 g/m²·day or lower at 5°C, 0.2 g/m²·day or lower at 25°C, and 0.2 g/m²·day or lower at 40°C, under test conditions according to the aforementioned ISO2528 or JIS K 7126-1:2006.

The material used for the needle cap of the present invention is required to have an elasticity to allow it to be attached to and removed from the syringe body and cohesively contact with the syringe body, and have a low water vapor permeability. Butyl rubbers (IIR) are specific examples.

The butyl rubbers include a n-butyl rubber as well as a halogenated butyl rubber such as a bromobutyl rubber (BIIR) or a chlorobutyl rubber (CIIR).

The structure of the cap is not specifically limited as long as the aforementioned material is formed as a tube with one end closed and the other end having an opening portion capable of being attached to a needle or a syringe body, sealing contact with its outer surface. It can have any of single-layered, multi-layered, and other structures. The cap can have a groove or a protrusion formed inside the opening portion for the attachment and removal of the cap. The cap can be attached so that it may cover from the tip of the needle to the tip of the outer cylinder which is the syringe body or from the tip of the needle to the connector between the needle and the syringe body.

The material of the syringe body of the syringe according to the present invention is not specifically limited and any material that can typically be used for syringes can be used. Specifically, a syringe made of glass or a resin can be used.

When the pre-filled syringe of the present invention is subjected to sterilization with radiation such as a gamma ray, the syringe body is preferably made of a material that is hardly affected (e.g., colored or degraded) by radiation. Specific examples include resins such as cycloolefinic resins, polyethylene resins, and polypropylene resins, and particularly preferable examples include cycloolefinic resins such as COPs (Cyclic Olefin Polymers: cycloolefinic polymers), COC (Cyclic Olefin Copolymers: cycloolefinic copolymers). Such syringes are particularly suitable for use in syringes for being filled by a tray filler system that is used for industrial mass productions.

The size of the volume (standard) of the syringe according to the present invention is not specifically limited. Specifically, an advantageous effect of the present invention is remarkably exhibited for small-volume syringes of 0.5 mL to 5.0 mL, preferably 1 mL.

The size of the needle is not specifically limited. Specifically, it is preferable that the outer diameter is 0.2 to 0.5 mm and the inner diameter is 0.1 to 0.3 mm. Typical needle gauges are: 25 (outer diameter of 0.50 to 0.53 mm), 26 (outer diameter of 0.44 to 0.47 mm), 27 (outer diameter of 0.40 to 0.42 mm), 28 (outer diameter of 0.34 to 0.37 mm), 29 (outer diameter of 0.32 to 0.35 mm), 30 (outer diameter of 0.29 to 0.32 mm), 31 (outer diameter of 0.25 to 0.27 mm), 32 (outer diameter of 0.22 to 0.24 mm) or 33G (outer diameter of 0.20 to 0.22 mm), but the present invention is not limited to these sizes.

The pre-filled syringe formulation of the present invention are typically those for self-injection. According to this configuration, if a user of the pre-filled syringe formulation is a patient rather than a health-care provider and the pre-filled syringe formulation of the present invention has been left in an inappropriate manner for a long time, it is possible to reduce the risk of causing clogging.

The degree of clogging is determined using a method described in the Examples below in the present invention.

For example, in the pre-filled syringe formulation of the present invention, no clogging occurs even after having been stored at 40°C and 8% RH for 4 weeks; or no clogging occurs even after having been stored at 40°C and 8% RH for 6 weeks; or no clogging occurs even after having been stored at 40°C and 8% RH for 2 months; or no clogging occurs even after having been stored at 40°C and 8% RH for 3 months; or no clogging occurs even after having been stored at 40°C and 8% RH for 4 months; or no clogging occurs even after having been stored at 40°C and 8% RH for 5 months; or no clogging occurs even after having been stored at 40°C and 8% RH for 6 months.

Furthermore, for example, in the pre-filled syringe formulation of the present invention, no clogging occurs even after having been stored at 25°C and 7% RH for 4 weeks; or no clogging occurs even after having been stored at 25°C and 7% RH for 6 weeks; or no clogging occurs even after having been stored at 25°C and 7% RH for 2 months; or no clogging occurs even after having been stored at 25°C and 7% RH for 2 months; or no clogging occurs even after having been stored at 25°C and 7% RH for 3 months; or no clogging occurs even after having been stored at 25°C and 7% RH for 4 months; or no clogging occurs even after having been stored at 25°C and 7% RH for 5 months; or no clogging occurs even after having been stored at 25°C and 7% RH for 6 months.

Moreover, for example, in the pre-filled syringe formulation of the present invention, no clogging occurs even after having been stored at 5°C and 25% RH for 1 months; or no clogging occurs even after having been stored at 5°C and 25% RH for 2 months; no clogging occurs even after having been stored at 5°C and 25% RH for 3 months; or no clogging occurs even after having been stored at 5°C and 25% RH for 6 months; or no clogging occurs even after having been stored at 5°C and 25% RH for 9 months; or no clogging occurs even after having been stored at 5°C and 25% RH for 12 months; or no clogging occurs even after having been stored at 5°C and 25% RH for 18 months; or no clogging occurs even after having been stored at 5°C and 25% RH for 24 months.

Next, manufacture of a pre-filled syringe formulation with staked needle with a needle cap according to another embodiment of the present invention is described.

In manufacturing pre-filled syringe formulation with staked needle with a needle cap, since pharmaceutical liquid is typically filled by a tray filler system, sterilization is performed before filling the pharmaceutical liquid; for example, it is performed in advance in a syringe manufacturer. To assemble a syringe with staked needle, a needle is attached to the tip of the syringe body and then a cap (i.e., a syringe cap or a needle cap) is attached so that it covers the needle. Next, the syringe with staked needle with the cap attached thereon is sterilized by irradiating radiation or an electron beam for a period sufficient to achieve sterilization. The most common way of radiation sterilization is a gamma-ray irradiation. Cobalt-60 is an example of a ray source used but the source is not limited thereto. Since the gamma ray is superior in its penetrating ability, it does not limit the form of packaging, has a small variation in dose, and can be used to sterilize a needle even with a cap made of a butyl rubber being placed on the needle. The dose of radiation depends on the amount of the object to be sterilized. Typically, a gamma ray is irradiated at an absorbed dose of about 10 kGy to 60 kGy, preferably about 25 kGy to 50 kGy. After the sterilization, the pharmaceutical liquid is filled in the syringe body and then a plunger is fitted, in an aseptic environment. A small number (e.g., one) of pre-filled syringe formulation with staked needle may then be packed in a pillow packs.

It is particularly preferable that the pre-filled syringe formulation with staked needle of the present invention are applied to a high-concentration protein solution for subcutaneous injection or the like. In the present invention, the protein solution refers to a formulated solution containing physiologically active protein as an active ingredient.

The concentration of the physiologically active protein in the protein solution is preferably 50 mg/ml or higher.

As the physiologically active protein, an antibody is preferable.

An antibody-containing formulated solution containing an antibody at a high concentration is particularly preferable.

In the present invention, the antibody-containing formulated solution refers to a formulated solution which contains an antibody as an active ingredient and which has been prepared so that it can be administered to animals such as human, and preferably refers to a formulated solution manufactured without lyophilization in the manufacturing process.

One embodiment of the present invention is a formulation for subcutaneous injection by self injection in which a formulated solution containing an antibody at a high concentration is filled in a pre-filled syringe with staked needle and the aforementioned syringe cap is removably attached to the aforementioned syringe body.

The formulated solution containing an antibody at a high concentration of the present invention refers to a solution having an antibody concentration of 50 mg/mL or higher, but preferably 80 mg/mL or higher, more preferably 100 mg/mL or higher, yet more preferably 120 mg/mL, yet more preferably 150 mg/mL.

In addition, the upper limit of the antibody concentration of the antibody-containing formulated solution according to the present invention is typically 300 mg/mL, preferably 250 mg/mL, and more preferably 200 mg/mL, from the manufacturing viewpoint. Accordingly, the antibody concentration of the formulated solution with an antibody at a high concentration according to the present invention is preferably 50 to 300 mg/mL, and 100 to 300 mg/mL is more preferable, 120 to 250 mg/mL is yet more preferable, and 150 to 200 mg/mL is particularly preferable.

Antibodies used in the present invention are not specifically limited as long as they are capable of binding to a target antigen. The antibodies may be polyclonal or monoclonal, but monoclonal antibodies are preferable since homogeneous antibodies can be stably produced.

Examples of the monoclonal antibodies used in the present invention include monoclonal antibodies derived from animals such as human, mice, rats, hamsters, rabbits, sheep, camels, and monkeys as well as recombinant antibodies that have been modified artificially, such as chimeric antibodies, humanized antibodies, and bispecific antibodies. In addition, the recombinant antibodies that have been modified artificially at, for example, the constant region to alter physical properties of antibody molecules (specifically, for example, alteration of an isoelectric point (pI) or alteration of the affinity for Fc receptors) for the purpose of improving retention in blood or pharmacokinetics, are also included.

Furthermore, immunoglobulin class of the antibodies used in the present invention is not specifically limited. Any of the classes IgG such as IgG1, IgG2, IgG3, and IgG4, IgA, IgD, IgE, and IgM can be used, but IgG and IgM are preferable.

In addition, examples of the antibodies used in the present invention include antibodies having the constant and variable regions (i.e., whole antibodies) as well as minibodies such as antibody fragments, e.g., Fv, Fab, and F(ab)₂, monovalent or divalent single-chain Fv (scFv, sc(Fv)2) in which variable regions of the antibody are linked by a linker such as a peptide linker, and diabodies, e.g., scFv dimers, but whole antibodies are preferable.

The aforementioned antibodies used in the present invention can be generated using a method widely known to those skilled in the art. Hybridomas that produce monoclonal antibodies can be generated basically using a known technique in a manner described below. Specifically, they can be generated by making immunization according to an ordinary immunization method using the target antigen or cells expressing the target antigen as a sensitizing antigen, fusing the immune cells obtained with known parental cells using an ordinary cell-fusion method, and screening the fused cells for monoclonal antibody-producing cells or hybridoma using an ordinary screening method. Hybridomas can be generated according to, for example, a method of Milstein et al. (Kohler. G. and Milstein, C., Methods Enzymol. (1981) 73:3-46). If the antigens have low immunogenicity, they can be conjugated to a macromolecule having immunogenicity, such as albumin to perform immunization.

Furthermore, recombinant antibodies can also be used, which are generated by cloning an antibody gene from a hybridoma, incorporating it into an appropriate vector, introducing it into host cells, producing antibodies using gene recombination techniques (see, for example, Carl, A. K. Borrebaeck, James, W. Larrick, THERAPEUTIC MONOCLONAL ANTIBODIES, Published in the United Kingdom by MACMILLAN PUBLISHERS LTD, 1990). Specifically, cDNA for the variable region (V-region) of an antibody is synthesized from mRNA of a hybridoma using a reverse transcriptase. Once DNA encoding the V-region of a target antibody is obtained, it is fused to DNA encoding the constant region (C-region) of a target antibody and is introduced into an expression vector. Alternatively, DNA encoding the V-region of an antibody can be incorporated into an expression vector having DNA for the C-region of an antibody. The fused DNA is incorporated into an expression vector to allow its expression under the regulation by a regulatory region such as an enhancer and a promoter. Next, the host cells are transformed with this expression vector to allow expression of the antibody.

In the present invention, recombinant antibodies that have been artificially modified, such as chimeric antibodies and humanized antibodies can be used for the purpose of reducing xenoantigenicity to human. These modified antibodies can be produced using known methods. Chimeric antibodies have the variable region of the heavy and light chains of an antibody from a non-human mammal such as mice and the constant region of the heavy and light chains of a human antibody. They can be obtained by ligating DNA encoding the variable region of the mouse antibody to DNA encoding the constant region of the human antibody, incorporating the fusion DNA into an expression vector, introducing it into a host and allowing the host to produce the product.

The humanized antibody is also referred to as a reshaped human antibody. It is obtained by transferring the complementarity determining region (CDR) of an antibody from a non-human mammal such as mice to CDR of a human antibody. General gene recombination techniques for this purpose are also known. Specifically, a recombinant antibody can be obtained by synthesizing DNA having a sequence on which the CDR of a mouse antibody and the framework region (FR) of a human antibody are designed to be fused, using the PCR method from a few oligonucleotides designed to have overlap regions at their terminals, ligating the DNA thus obtained to DNA encoding the constant region of a human antibody, incorporating the construct into an expression vector and then introducing the vector into a host to allow the host to produce the product (see, EP 239400 and WO 96/02576). The FR of a human antibody which is linked via CDR is selected based on the formation of a good antigen-binding site by the complementarity determining region. Optionally, the amino acids of the framework region of the variable region of an antibody may be substituted so that the complementarity determining region of a reshaped human antibody can form an appropriate antigen-binding site (Sato, K. et al., Cancer Res. (1993) 53, 851-856).

As examples of techniques to substitute amino acids of antibodies to improve, for example, the activity, physical properties, pharmacokinetics, and safety of the antibodies, those described below are known. Antibodies used in the present invention include those subjected to such substitution (including deletion and addition) of amino acids.

Techniques for amino acid substitutions in the variable region of IgG antibodies have been reported which include humanization (Tsurushita N, Hinton PR, Kumar S., Design of humanized antibodies: from anti-Tac to Zenapax., Methods. 2005 May; 36(1):69-83) as well as affinity maturation by substitution of amino acids in the complementarity determining region (CDR) for enhancing the binding activity (Rajpal A, Beyaz N, Haber L, Cappuccilli G, Yee H, Bhatt RR, Takeuchi T, Lerner RA, Crea R., A general method for greatly improving the affinity of antibodies by using combinatorial libraries., Proc Natl Acad Sci USA. 2005 Jun 14; 102(24):8466-71) and improvement of the physicochemical stability by substitution of amino acids in the framework region (FR) (Ewert S, Honegger A, Pluckthun A., Stability improvement of antibodies for extracellular and intracellular applications: CDR grafting to stable frameworks and structure-based framework engineering., Methods. 2004 Oct; 34(2):184-99. Review). In addition, as techniques for making amino acid substitutions in the Fc region of IgG antibodies, those of enhancing antibody-dependent cellular cytotoxicity (ADCC) and complement-dependent cytotoxicity (CDC) are known (Kim SJ, Park Y, Hong HJ., Antibody engineering for the development of therapeutic antibodies., Mol Cells. 2005 Aug 31; 20(1):17-29. Review). Furthermore, besides techniques of enhancing such effector functions, those for substituting amino acids in Fc to improve the antibody half-life in blood are reported (Hinton PR, Xiong JM, Johlfs MG, Tang MT, Keller S, Tsurushita N., An engineered human IgG1 antibody with longer serum half-life., J Immunol. 2006 Jan 1; 176(1):346-56, Ghetie V, Popov S, Borvak J, Radu C, Matesoi D, Medesan C, Ober RJ, Ward ES., Increasing the serum persistence of an IgG fragment by random mutagenesis., Nat Biotechnol. 1997 Jul;15(7):637-40). Furthermore, various techniques for substituting amino acids in the constant regions for the purpose of improving the physical properties of an antibody are also known (WO 09/41613).

In addition, methods for obtaining human antibodies are also known. For example, desired human antibodies with binding activity to a target antigen can be obtained by stimulating human lymphocytes with the antigen or cells expressing the antigen *in vitro* and fusing the stimulated lymphocytes with human myeloma cells such as U266 (see, Japanese Patent Publication No. 1-59878(B)). The desired human antibodies can be also obtained by immunizing transgenic animals having the entire repertoire of human antibody genes with an antigen (see, WO 93/12227, WO 92/03918, WO 94/02602, WO 94/25585, WO 96/34096, and WO 96/33735). Furthermore, techniques of obtaining human antibodies by panning using a human antibody library are known. For example, the variable regions of human antibodies can be expressed as single-chain antibodies (scFvs) on the surface of phages using a phage display method, and then phages that bind to the antigen can be selected. The genes of the selected phages can be analyzed to determine DNA sequences capable of encoding the variable regions of human antibodies that bind to the antigen. Once the DNA sequences of scFvs that bind to the antigen are identified, appropriate expression vectors carrying these sequences can be constructed to obtain human antibodies. Such methods are already widely known, referring to WO 92/01047, WO 92/20791, WO 93/06213, WO 93/11236, WO 93/19172, WO 95/01438, and WO 95/15388. The antibodies used in the present invention also include such human antibodies.

When the antibody genes are isolated and introduced into appropriate hosts to produce antibodies, appropriate combinations of hosts and expression vectors can be used. When eukaryotic cells are used as a host, animal cells, plant cells, and fungal cells can be used. As animal cells, (1) mammalian cells such as CHO, COS, myeloma, baby hamster kidney (BHK), HeLa, and Vero; (2) amphibian cells such as Xenopus oocytes; and (3) insect cells such as sf9, sf21, and Tn5 are known. As plant cells, those derived from genus *Nicotiana* such as *Nicotiana tabacum* are known, which can be cultured as a callus. As fungal cells, yeasts such as genus *Saccharomyces,* e.g., *Saccharomyces cerevisiae,* and filamentous fungi such as genus *Aspergillus,* e.g., *Aspergillus niger* are known. When prokaryotic cells are used, production systems using bacterial cells are available. As bacterial cells, *Escherichia coli (E. coli)* and *Bacillus subtilis* are known. The desired antibodies can be obtained by introducing the genes encoding the antibodies into these cells by transformation and culturing the transformed cells in vitro.

Furthermore, the antibodies used in the present invention include modified antibodies. For example, antibodies linked to polyethylene glycol (PEG) or various molecules such as cytotoxic agents can be used (Farmaco. 1999 Aug 30; 54(8):497-516, Cancer J. 2008 May-Jun; 14(3):154-69). The antibodies used in the present invention also include such modified antibodies. Such modified antibodies can be prepared by chemically modifying the antibodies. Such methods have already been established in this field.

Examples of the antibodies used in the present invention include, but are not limited to, anti-tissue factor antibodies, anti-IL-6 receptor antibodies, anti-IL-6 antibodies, anti-glypican-3 antibodies, anti-CD3 antibodies, anti-CD20 antibodies, anti-GPIIb/IIIa antibodies, anti-TNF antibodies, anti-CD25 antibodies, anti-EGFR antibodies, anti-Her2/neu antibodies, anti-RSV antibodies, anti-CD33 antibodies, anti-CD52 antibodies, anti-IgE antibodies, anti-CD11a antibodies, anti-VEGF antibodies, anti-VLA4 antibodies, anti-HM1.24 antigen antibodies, anti-parathyroid hormone-related peptide antibodies (anti-PTHrP antibodies), anti-ganglioside GM3 antibodies, anti-TPO receptor agonist antibodies, antibodies as a functional substitute for coagulation factor VIII, anti-IL31 receptor antibodies, anti-HLA antibodies, anti-AXL antibodies, anti-CXCR4 antibodies, anti-NR10 antibodies, and bispecific antibodies against factor IX and factor X.

Examples of preferred reshaped human antibodies used in the present invention include humanized anti-interleukin 6 (IL-6) receptor antibodies (tocilizumab, hPM-1, and MRA; see, WO92/19759), humanized anti-HM1.24 antigen monoclonal antibodies (see, WO98/14580), humanized anti-parathyroid hormone-related peptide antibodies (anti-PTHrP antibodies) (see, WO98/13388), humanized anti-tissue factor antibodies (see, WO99/51743), humanized anti-glypican-3 IgG1 kappa antibodies (codrituzumab and GC33; see, WO2006/006693), anti-NR10 humanized antibodies (see, WO2009/072604), and bispecific humanized antibodies against factor IX and factor X (ACE910; see, WO2012/067176). Particularly preferred humanized antibodies used in the present invention are humanized anti-IL-6 receptor antibodies, anti-NR10 humanized antibodies, and bispecific humanized antibodies against factor IX and factor X.

As human IgM antibodies, preferable examples include recombinant human anti-ganglioside GM3 IgM antibodies (see, WO05/05636).

As minibodies, preferable examples include anti-TPO receptor agonist diabodies (see, WO02/33072 and anti-CD47 agonist diabodies (see, WO01/66737).

In the present invention, antibodies whose isoelectric point is low (low-pI antibodies) refers especially to antibodies with such a low isoelectric point that the antibodies in nature hardly have. The isoelectric point of such antibodies is, for example, 3.0 to 8.0, preferably 5.0 to 7.5, more preferably 5.0 to 7.0, and particularly preferably 5.0 to 6.5, but not limited thereto. In general, natural (or ordinary) antibodies are assumed to have an isoelectric point within a range of 7.5 to 9.5.

Furthermore, as the antibodies used in the present invention, pI-modified antibodies whose pI has been lowered by modifying surface amino acid residues of the antibodies are preferable. Such pI-modified antibodies refer to those whose pI has been lowered than that of the pre-modified antibody by one or more, preferably two or more, and more preferably three or more. Examples of pI-modified antibodies include, but not limited to SA237 (MAbI, H chain /SEQ ID NO: 1; L chain/SEQ ID NO: 2), which is an anti-IL-6 receptor antibody described in WO 2009/041621, and fully humanized NS22 antibodies, which are anti-NR10 humanized antibodies, produced by the method described in Example 12 of WO02009/072604.

In the case of H chain variable regions, surface amino acid residues are selected from, but not limited to, amino acid residues H1, H3, H5, H8, H10, H12, H13, H15, H16, H19, H23, H25, H26, H31, H39, H42, H43, H44, H46, H61, H62, H64, H65, H68, H71, H72, H73, H75, H76, H81, H82b, H83, H85, H86, H105, H108, H110, and H112, according to the Kabat numbering system. In the case of L chain variable regions, surface amino acid residues are selected from, but not limited to, amino acid residues L1, L3, L7, L8, L9, L11, L12, L16, L17, L18, L20, L22, L24, L27, L38, L39, L41, L42, L43, L45, L46, L49, L53, L54, L55, L57, L60, L63, L65, L66, L68, L69, L70, L74, L76, L77, L79, L80, L81, L85, L100, L103, L105, L106, and L107, according to the Kabat numbering system.

In the present invention, "modification" refers to, for example, substitution of an original amino acid residue with another amino acid residue, deletion of an original amino acid residue, and addition of a new amino acid residue. It, however, preferably refers to substitution of an original amino acid residue with another amino acid residue.

It is known that some amino acids are charge-bearing. In general, lysine (K), arginine (R), and histidine (H) are known as positively charged amino acids. Aspartic acid (D) and glutamic acid (E) are known as negatively charged amino acids. Other amino acids are known as uncharged amino acids.

In the present invention, modified amino acid residues are preferably selected from the amino acid residues included in the following groups (a) or (b) but not limited to these amino acids:
(a) gluramic acid (E) and aspartic acid (D),
(b) lysine (K), arginine (R), and histidine (H).

When a pre-modified amino acid residue has already electrically charged, modification into an uncharged amino acid residue is also a preferred embodiment.

Thus, the modification in the present invention includes (1) substitution of a charged amino acid with an uncharged amino acid, (2) substitution of a charged amino acid with an oppositely charged amino acid, and (3) substitution of an uncharged amino acid with a charged amino acid.

The value of the isoelectric point can be determined by isoelectric focusing, which is known to those skilled in the art. The value of the theoretical isoelectric point can be calculated using gene and amino acid sequence analysis software (e.g., Genetyx).

The antibodies having amino acid residues with their charge modified can be obtained by modifying a nucleic acid encoding an antibody, culturing the nucleic acid in host cells, and purifying the antibody from the culture of the host cells. In the present invention, the phrase "modify(ing) nucleic acids" refers to modifying nucleic acid sequences so that they have codons corresponding to amino acid residues introduced by the modifications. More specifically, it refers to modifying the nucleotide sequences of nucleic acids so that codons of the pre-modified amino acid residues are modified to those of the amino acid residues that are to be introduced by the modification. In other words, codons encoding the original amino acid residues are substituted with those encoding the amino acid residues that are to be introduced by the modification. Such nucleic acid modifications can be suitably performed by those skilled in the art using known techniques such as site-directed mutagenesis and PCR mutagenesis.

A buffer used for the protein-containing formulated solution of the present invention is prepared using a buffering agent which is a substance for maintaining a pH of the solution. In the formulated solution containing an antibody at a high concentration of the present invention, a pH of the solution is preferably 4 to 8, more preferably 5.0 to 7.5, still more preferably 5.5 to 7.2, and still more preferably 6.0 to 6.5. Buffering agents with which pH can be adjusted in this range and which are pharmaceutically acceptable can be used in the present invention. Such buffering agents are known by those skilled in the field of the formulated solution, and examples thereof include inorganic salts such as phosphates (sodium or potassium) and sodium hydrogen carbonate; organic acid salts such as citrates (sodium or potassium), sodium acetate, and sodium succinate; and acids such as phosphoric acid, carbonic acid, citric acid, succinic acid, malic acid and gluconic acid. Furthermore, Tris, Good's buffering agents such as MES, MOPS and HEPES, histidine (e.g., histidine hydrochloride) and glycine can also be used.

In the formulated solution containing an antibody at a high concentration of the present invention, the buffer is preferably a histidine buffer or a glycine buffer, and a histidine buffer is particularly preferred. The concentration of the buffer is typically 1 to 500 mM, preferably 5 to 100 mM, and more preferably 10 to 20 mM. When a histidine buffer is used, the buffer contains histidine at a concentration of preferably 5 to 25 mM, more preferably 10 to 20 mM.

It is preferable that the formulated solution containing an antibody at a high concentration according to the present invention is stabilized by adding a stabilizing agent that is suitable for the antibody which is an active ingredient. In the "stable" formulated solution containing an antibody at a high concentration according to the present invention, no significant change is observed when it is stored at a refrigeration temperature (2 to 8°C) for at least 12 months, preferably for 2 years, and more preferably for 3 years; or when it is stored at room temperature (22 to 28°C) for at least 3 months, preferably 6 months, and more preferably 1 year. For example, the total amount of dimers and degradation products in the formulated solution when it is stored at 5°C for 2 years is 5.0% or less, preferably 2% or less, and more preferably 1.5% or less; or the total amount of dimers and degradation products in the formulated solution when it is stored at 25°C for 6 months is 5.0% or less, preferably 2% or less, and more preferably 1.5% or less.

The preparation of the present invention can further contain a surfactant.

Typical examples of the surfactant include nonionic surfactants, for example, sorbitan fatty acid esters such as sorbitan monocaprylate, sorbitan monolaurate and sorbitan monopalmitate; glycerin fatty acid esters such as glycerol monocaprylate, glycerol monomyristate and glycerol monostearate; polyglycerol fatty acid esters such as decaglyceryl monostearate, decaglyceryl distearate and decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate and polyoxyethylene sorbitan tristearate; polyoxyethylene sorbitol fatty acid esters such as polyoxyethylene sorbitol tetrastearate and polyoxyethylene sorbitol tetraoleate; polyoxyethylene glycerol fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol ether, polyoxyethylene polyoxypropylene propyl ether and polyoxyethylene polyoxypropylene cetyl ether; polyoxyethylene alkyl phenyl ethers such as polyoxyethylene nonylphenyl ether; polyoxyethylene hardened castor oils such as polyoxyethylene castor oil and polyoxyethylene hardened castor oil (polyoxyethylene hydrogenated castor oil); polyoxyethylene bees wax derivatives such as polyoxyethylene sorbitol bees wax; polyoxyethylene lanolin derivatives such as polyoxyethylene lanolin; surfactants having an HLB of 6 to 18 such as polyoxyethylene fatty acid amides, for example, polyoxyethylene stearamide; anionic surfactants, for example, alkyl sulfate salts having an alkyl group with 10 to 18 carbon atoms, such as sodium cetyl sulfate, sodium lauryl sulfate and sodium oleyl sulfate; polyoxyethylene alkyl ether sulfate salts in which the average number of moles of the added ethylene oxide units is 2 to 4 and the number of carbon atoms of the alkyl group is 10 to 18, such as polyoxyethylene sodium lauryl sulfate; alkyl sulfosuccinate salts having an alkyl group with 8 to 18 carbon atoms, such as lauryl sulfosuccinate sodium salt; natural surfactants such as lecithin and glycerophospholipids; sphingophospholipids such as sphingomyelin; and sucrose esters of fatty acids with 12 to 18 carbon atoms. These surfactants can be added to the formulation of the present invention singly or in combination of two or more.

Preferred surfactants are polyoxyethylene sorbitan fatty acid esters and polyoxyethylene polyoxypropylene alkyl ethers. Polysorbates 20, 21, 40, 60, 65, 80, 81 and 85 and Pluronic type surfactants are particularly preferable, and polysorbates 20 and 80 and Pluronic F-68 (Poloxamer 188) are most preferable.

The amount of the surfactant(s) to be added to the antibody-containing preparation according to the present invention is typically 0.0001 to 10% (w/v), preferably 0.001 to 5%, more preferably 0.005 to 3%.

Optionally, suspending agents, solubilizing agents, isotonizing agents, preservatives, adsorption inhibitors, diluents, excipients, pH adjustors, analgesics, sulfur-containing reducing agents, antioxidants, and the like can appropriately be added to the preparation of the present invention.

Examples of the suspending agent include methyl cellulose, polysorbate 80, hydroxyethyl cellulose, gum arabic, powdered tragacanth, sodium carboxymethylcellulose, and polyoxyethylene sorbitan monolaurate.

Examples of the solubilizing agents include polyoxyethylene hydrogenated castor oil, polysorbate 80, nicotinamide, polyoxyethylene sorbitan monolaurate, macrogol, and castor oil fatty acid ethyl ester.

Examples of the isotonizing agents include sodium chloride, potassium chloride, and calcium chloride.

Examples of the preservatives include methyl parahydroxybenzoate, ethyl parahydroxybenzoate, sorbic acid, phenol, cresol, and chlorocresol.

Examples of the adsorption inhibitors include human serum albumin, lecithin, dextran, ethylene oxide/propylene oxide copolymers, hydroxypropyl cellulose, methyl cellulose, polyoxyethylene hydrogenated castor oil, and polyethylene glycol.

Examples of the sulfur-containing reducing agents include those containing sulfhydryl groups such as N-acetylcysteine, N-acetylhomocysteine, thioctic acid, thiodiglycol, thioethanol amine, thioglycerol, thiosorbitol, thioglycolic acid and salts thereof, sodium thiosulfate, glutathione, and thioalkanoic acids having one to seven carbon atoms.

Examples of the antioxidants include erythorbic acid, dibutylhydroxytoluene, butylhydroxyanisole, alpha-tocopherol, tocopherol acetate, L-ascorbic acid and salts thereof, L-ascorbic acid palmitate, L-ascorbic acid stearate, sodium hydrogen sulfite, sodium sulfite, triamyl gallate, propyl gallate, and chelating agents such as disodium ethylenediamine tetraacetate (EDTA), sodium pyrophosphate, and sodium metaphosphate.

Antibody-containing formulated solutions in a pre-filled syringe of the present invention are administered by, for example, subcutaneous, intravenous, or intramuscular injection. Since the amount of antibody for a single dose is large (i.e., about 80 to 200 mg) but subcutaneous injections have a limitation on the volume of the liquid to be injected, the formulated solution of the present invention is particularly suitable for subcutaneous injection.

The osmotic pressure ratio of the antibody-containing formulated solution according to the present invention is preferably about 0.5 to 4, more preferably about 0.7 to 2, and still more preferably about 1.

The viscosity of the antibody-containing formulated solution according to the present invention is preferably about 2 to 100 mPa·s, more preferably about 2 to 50 mPa·s, still more preferably about 4 to 50 mPa·s, and still more preferably about 6 to 50 mPa·s. It should be noted that the viscosity described herein is measured by a rotation viscometer method using a cone-plate type viscometer (2.53 Viscosity Determination/General Tests, the Japanese Pharmacopoeia, 15th edition).

As can be seen from the examples of the present application, when the viscosity of the solution containing a protein such as an antibody is 6 mPa·s or higher, preferably 12 mPa·s or higher, more preferably 20 mPa·s or higher, still more preferably 50mPa·s or higher, it was found that clogging occurs in a short period of time (within 10 minutes) after the cap was removed from the needle of the syringe. Accordingly, when the viscosity of the protein solution of the present invention is 6 to 100 mPa·s, suitably 12 to 100 mPa·s, more suitably 20 to 100 mPa·s, still more suitably 50 to 100 mPa·s, the syringe cap of the present invention is essential.

Even with the same protein concentration, the viscosity of the formulated solution can vary depending on its ingredient(s) other than the antibody in the formulated solution. The risk of clogging increases with the increase of the viscosity. Embodiments of formulated solutions with an antibody at a high concentration which are filled in pre-filled syringes of the present invention and sealed include formulated solutions with: the antibody concentration of 100 to 300 mg/mL and the viscosity of 6 to 100 mPa·s; the antibody concentration of 100 to 300 mg/mL and the viscosity of 12 to 100 mPa·s; the antibody concentration of 100 to 300 mg/mL and the viscosity of 20 to 100 mPa·s; the antibody concentration of 100 to 300 mg/mL and the viscosity of 50 to 100 mPa·s; the antibody concentration of 120 to 250 mg/mL and the viscosity of 6 to 100 mPa·s; the antibody concentration of 120 to 250 mg/mL and the viscosity of 12 to 100 mPa·s; the antibody concentration of 120 to 250 mg/mL and the viscosity of 20 to 100 mPa·s; the antibody concentration of 120 to 250 mg/mL and the viscosity of 50 to 100 mPa·s; the antibody concentration of 150 to 200 mg/mL and the viscosity of 6 to 100 mPa·s; the antibody concentration of 150 to 200 mg/mL and the viscosity of 12 to 100 mPa·s; the antibody concentration of 150 to 200 mg/mL and the viscosity of 20 to 100 mPa·s; and the antibody concentration of 150 to 200 mg/mL and the viscosity of 50 to 100 mPa·s.

In the formulated solution containing an antibody at a high concentration used in the present invention, the antibody is preferably a humanized antibody or an anti-interleukin-6 receptor antibody. The humanized anti-interleukin-6 receptor antibody is preferably tocilizumab.

An example of the formulated solution containing humanized anti-interleukin-6 receptor antibody as an active ingredient at a high concentration is Actemra solution for subcutaneous injection used in the Examples described below. For Actemra solution for subcutaneous injection, a subcutaneous injection highly concentrated to 180 mg/mL has been approved and a self-injectable pre-filled syringe formulation is commercially available which contains, in a syringe of 0.9 mL in volume with staked needle, 162 mg of antibody (tocilizumab (recombinant)) as an active ingredient, polysorbate 80 as a surfactant, and arginine and methionine as stabilizing agents. A syringe body in which the pharmaceutical liquid is filled, however, is made of glass, and its syringe cap is made of an isoprene rubber having gas permeability to enable gas sterilization. Accordingly, a material having a low gas permeability is used for a pillow film to prevent evaporation of the pharmaceutical liquid.

The present invention is described more in detail below in the following Examples, but the scope of the present invention is not limited thereto.

### EXAMPLES

### EXAMPLE 1

COP syringes (1 ml standard) having a 27G needle, which had been sterilized using radiation (25 kGy) with a rubber cap (made of a chlorobutyl rubber) having an extremely low moisture permeability thereon, were aseptically filled with 0.9 ml of antibody-containing solution (tocilizumab: 180 mg/mL, buffer: 20 mmol/L of histidine, stabilizers: 100 mmol/L of arginine and 30 mmol/L of methionine, surfactant: 0.2 mg/mL of polysorbate 80, pH 6.0, viscosity: about 8 mPa/s), and plugged using a stopper, which were stored at a low humidity (till 6 months at 40°C; till 6 months at 25°C; and till 24 months at 5°C). Thereafter, clogging was evaluated.

As controls, glass syringes (1 ml standard) having a 27G needle, which had been sterilized using gas with a rubber cap (made of isoprene) having a moisture permeability thereon and filled with 0.9 ml of antibody-containing solution in a similar manner, were used.

### Method of evaluation of clogging

Each sample was placed at a specified position in an autograph. Syringes with staked needle fixed in advance to the autograph were operated for discharge. The load exerted on the plunger stopper was measured at a discharge rate of 100 mm/min. For the definition of the clogging, if the sample was not discharged or a load stress was abnormally high as compared with normal discharge, that sample was judged as being clogged.

### Results of evaluation

It was found that no clogging occurred as shown in the following table after the syringes had been stored at a low humidity (till 6 months at 40°C; till 6 months at 25°C; and till 24 months at 5°C). On the other hand, clogging was observed in all cases (3/3) after having been stored for 4 weeks at 40°C and 8% RH when a rubber cap having moisture permeability was used.

**[Table 1]**

| Storage condition | 2week | 4week | 6week | 2M | 2.5M | 3M | 4M | 5M | 6M | |
|---|---|---|---|---|---|---|---|---|---|---|
| 40°C-8%RH | 0% | 0% | 0% | 0% | 0% | 0% | 0% | 0% | 0% | |
| | (0/5) | (0/5) | (0/5) | (0/5) | (0/5) | (0/5) | (0/5) | (0/5) | (0/5) | |
| 25°C-7%RH | 0% | 0% | 0% | 0% | 0% | 0% | 0% | 0% | 0% | |
| | (0/5) | (0/5) | (0/5) | (0/5) | (0/5) | (0/5) | (0/5) | (0/5) | (0/5) | |

| Storage condition | 1M | 1.5M | 2M | 2.5M | 3M | 6M | 9M | 12M | 18M | 24M |
|---|---|---|---|---|---|---|---|---|---|---|
| 5°C-25%RH | 0% | 0% | 0% | 0% | 0% | 0% | 0% | 0% | 0% | 0% |
| | (0/5) | (0/5) | (0/5) | (0/5) | (0/5) | (0/5) | (0/5) | (0/5) | (0/3) | (0/3) |

| | | | | | | | | | | |
|---|---|---|---|---|---|---|---|---|---|---|
| %: Percentage of clogging (Number of clogged syringe formulation/Number of assessed syringe formulation) | | | | | | | | | | |

### EXAMPLE 2

To examine a possibility that a frequency of occurrence of clogging may vary depending on the viscosity even with the same concentration of protein, two different antibody-containing solutions having the same protein concentration and different viscosities were prepared and frequency of occurrence of clogging were compared with each other. The antibody (Mab1) used was an anti-IL-6 receptor antibody described in WO 2009/041621, which was also called Mab1 in WO2011/090088. Amino acid sequences of the antibody are represented by SEQ ID NOs. 1 and 2 for the H and L chains, respectively, which have been described in WO2011/090088.

The concentration of the antibody was 180 mg/mL, and the formulations were as follows: 20 mmol/L of histidine, 140 mmol/L of arginine, appropriate amount of aspartic acid, pH 6.0 (sample A) or 20 mmol/L of histidine, 20 mmol/L of arginine, appropriate amount of aspartic acid and hydrochloric acid, pH 6.0 (sample B).

### Method of evaluation of viscosity

Viscosities *η*(mPa·s) of the samples were measured using an EMS viscometer (Kyotodenshi) (J Artif Organs. 16:359-367 (2013)). The experiments were performed at 25°C. The results of the measurement are given in Table 2.

### Method of evaluation of clogging

COP syringes (1 ml standard) having a 27G needle were filled with 1.0 ml of antibody-containing solution and plugged with a stopper. The samples left at room temperature for 0, 10, 30, and 60 minutes with the needle filled with the antibody-containing solution without a rubber cap were placed at a specified position in an autograph and a load exerted on the plunger stopper was measured at a discharge rate of 100 mm/min. For the definition of the clogging, if the sample was not discharged or a load stress was abnormally high as compared with normal discharge, that sample was judged as being clogged. The measurement was performed with N = 3 at each time point and frequencies of occurrence of clogging were calculated. The results of the measurement are given in Table 2.

### Results of evaluation

It was found that even when the protein concentration is the same, the risk of clogging increased with the increase of the viscosity.

**[Table 2]**

| | Formulation | Viscosity *η* (mPa·s) | Frequency of occurrence of clogging | | | |
|---|---|---|---|---|---|---|
| | | | 0 min. | 10 min. | 30 min. | 60 min. |
| Sample A | 180 mg/mL Mab1, 20 mmol/L histidine, 140 mmol/L arginine, appropriate amount of aspartic acid, pH 6.0 | 14.7 | 0% | 67% | 33% | 33% |
| | | | (0/3) | (2/3) | (1/3) | (1/3) |
| Sample B | 180 mg/mL Mab1, 20 mmol/L histidine, 20 mmol/L arginine, appropriate amount of aspartic acid and hydrochloric acid, pH 6.0 | 54.8 | 0/3 | 100% | 100% | 100% |
| | | | (0/3) | (3/3) | (3/3) | (3/3) |

| | | | | | | |
|---|---|---|---|---|---|---|
| %: Percentage of clogging (Number of clogged syringe formulation/Number of assessed syringe formulation) | | | | | | |

## Claims

1. A pre-filled syringe formulation with staked needle, the pre-filled syringe formulation being filled with a protein solution and sealed, comprising:
a cap on the needle, the cap being made of a material having a low water vapor permeability.

2. The pre-filled syringe formulation according to Claim 1, wherein the material of the cap is a butyl rubber.

3. The pre-filled syringe formulation according to Claim 2, wherein the butyl rubber is a n-butyl rubber or a halogenated butyl rubber.

4. The pre-filled syringe formulation according to any one of Claims 1 to 3, wherein a syringe body is made of a resin.

5. The pre-filled syringe formulation according to Claim 4, wherein the syringe body is made of a cycloolefinic resin.

6. The pre-filled syringe formulation according to Claim 5, wherein the cycloolefinic resin is COP or COC.

7. The pre-filled syringe formulation according to any one of Claims 1 to 6, wherein the protein solution comprises a protein at 80 mg/ml or more.

8. The pre-filled syringe formulation according to Claim 7, wherein the protein solution comprises the protein at 100 mg/ml or more.

9. The pre-filled syringe formulation according to any one of Claims 1 to 8, wherein the protein solution has a viscosity of 2 to 100 mPa· s.

10. The pre-filled syringe formulation according to any one of Claims 1 to 9, wherein the protein is an antibody.

11. The pre-filled syringe formulation according to Claim 10, wherein a concentration of the antibody is 100 to 300 mg/mL, and the viscosity is 6 to 100m Pa·s.

12. The pre-filled syringe formulation according to Claim 10 or 11, wherein the antibody is an anti-IL6 receptor antibody.

13. The pre-filled syringe formulation according to Claim 12, wherein the antibody is tocilizumab.

14. A method of producing a pre-filled syringe formulation with staked needle, the pre-filled syringe formulation being filled with a protein solution and sealed, the method comprising the steps of:
1) sterilizing a syringe with staked needle with radiation or an electron beam, the syringe comprising a cap made of a material having a low water vapor permeability; and
2) filling, with a protein solution, and sealing the syringe with staked needle aseptically.


SEQUENCE LISTING

<110>  CHUGAI SEIYAKU KABUSHIKI KAISHA

<120>  A needle-tipped prefilled-syringe composition, with a cap covering
the needle

<130>  FA0001-15173

<150>  JP 2014-221127
<151>  2014-10-30

<160>  2

<170>  PatentIn version 3.4

<210>  1
<211>  443
<212>  PRT
<213>  Artificial

<220>
<223>  An artificially synthesized peptide sequence

<400>  1

Gln Val Gln Leu Gln Glu Ser Gly Pro Gly Leu Val Lys Pro Ser Glu
1               5                   10                  15

Thr Leu Ser Leu Thr Cys Ala Val Ser Gly His Ser Ile Ser His Asp
            20                  25                  30

His Ala Trp Ser Trp Val Arg Gln Pro Pro Gly Glu Gly Leu Glu Trp
        35                  40                  45

Ile Gly Phe Ile Ser Tyr Ser Gly Ile Thr Asn Tyr Asn Pro Ser Leu
    50                  55                  60

Gln Gly Arg Val Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Leu Ala Arg Thr Thr Ala Met Asp Tyr Trp Gly Glu Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Asn Phe Gly Thr Gln Thr Tyr Thr Cys Asn Val Asp His Lys Pro
        195                 200                 205

Ser Asn Thr Lys Val Asp Lys Thr Val Glu Arg Lys Ser Cys Val Glu
    210                 215                 220

Cys Pro Pro Cys Pro Ala Pro Pro Val Ala Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Phe Arg Val Val Ser Val Leu
    290                 295                 300

Thr Val Val His Gln Asp Trp Leu Asn Gly Lys Glu Tyr Lys Cys Lys
305                 310                 315                 320

Val Ser Asn Lys Gly Leu Pro Ala Pro Ile Glu Lys Thr Ile Ser Lys
                325                 330                 335

Thr Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Met Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Ala
            420                 425                 430

His Tyr Thr Gln Lys Ser Leu Ser Leu Ser Pro
        435                 440

<210> 2
<211> 214
<212> PRT
<213> Artificial

<220>
<223> An artificially synthesized peptide sequence

<400> 2

Asp Ile Gln Met Thr Gln Ser Pro Ser Ser Leu Ser Ala Ser Val Gly
1               5                   10                  15

Asp Ser Val Thr Ile Thr Cys Gln Ala Ser Thr Asp Ile Ser Ser His
            20                  25                  30

Leu Asn Trp Tyr Gln Gln Lys Pro Gly Lys Ala Pro Glu Leu Leu Ile
        35                  40                  45

Tyr Tyr Gly Ser His Leu Leu Ser Gly Val Pro Ser Arg Phe Ser Gly
    50                  55                  60

Ser Gly Ser Gly Thr Asp Phe Thr Phe Thr Ile Ser Ser Leu Glu Ala
65                  70                  75                  80

Glu Asp Ala Ala Thr Tyr Tyr Cys Gly Gln Gly Asn Arg Leu Pro Tyr
                85                  90                  95

Thr Phe Gly Gln Gly Thr Lys Val Glu Ile Glu Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210